Europäisches Patentamt

European Patent Office

Office européen des brevets

(19)

(11) Publication number: **0 025 745**
**B1**

(12)

# EUROPEAN PATENT SPECIFICATION

(45) Date of publication of patent specification: **11.07.84**

(51) Int. Cl.³: **C 12 N 7/08**

(21) Application number: **80401258.1**

(22) Date of filing: **03.09.80**

(54) **Process for propagating human hepatitis A virus in in vitro cell culture.**

(30) Priority: **04.09.79 US 71648**

(43) Date of publication of application:
**25.03.81 Bulletin 81/12**

(45) Publication of the grant of the patent:
**11.07.84 Bulletin 84/28**

(84) Designated Contracting States:
**AT BE CH DE FR GB IT LI LU NL SE**

(56) References cited:
**FR - A - 2 398 504**
**GB - A - 811 411**
**GB - A - 878 704**
**US - A - 3 871 954**
**US - A - 3 935 066**
**US - A - 4 164 566**

(73) Proprietor: **MERCK & CO. INC.**
**126, East Lincoln Avenue P.O. Box 2000**
**Rahway New Jersey 07065 (US)**

(72) Inventor: **Provost, Philip J.**
**473 Landis Road**
**Harleysville Pennsylvania 19438 (US)**
Inventor: **Giesa, Paula A.**
**1708 Lincoln Drive East**
**Ambler Pennsylvania 19002 (US)**
Inventor: **Hilleman, Maurice R.**
**4107 Fields Drive**
**Lafayette Hill Pennsylvania 19444 (US)**

(74) Representative: **Corre, Jacques Denis Paul et al,**
**Cabinet Regimbeau 26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

Prior art methods for obtaining hepatitis A antigen as disclosed by Provost et al. in U.S. patent 4,029,764 involved intravenous inoculation of a non-human primate with heptatitis A virus with subsequent removal of the infected liver and recovery of virus therefrom. Another method disclosed by Provost et al. in U.S. patent application Serial No. 934,293 filed 17 August 1978, now U.S. patent 4,164,566, involves one or more passages of hepatitis A virus in a sub-human primate, removing infected liver tissue and using such tissue to inoculate an *in vitro* cell culture, incubating the cell culture until hepatitis A antigen is detectable, and carrying out at least one additional *in vitro* cell culture passage of the virus. A disadvantage of these processes is that they require the use of primates that are not only expensive but in short supply and difficult to obtain. An *in vitro* cell culture system which did not require the use of primates would be a significant advance in this art.

### Objects of the Invention

It is an object of the present invention to provide an improved method for the *in vitro* cell culture of hepatitis A virus. Another object is to provide a method for propagation of hepatitis A virus which does not require the use of primates. A further object is to provide a method for the preparation of hepatitis A antigen for use in human vaccines and as a diagnostic antigen. Still another object is to provide an *in vitro* method for attenuating the virulence of hepatitis A antigen. These and other objects of the present invention will be apparent from the following description.

### Summary of the Invention

Hepatitis A virus is propagated in cell culture *in vitro* by direct inoculation of the cell cultures with human clinical specimens containing the virus.

### Detailed Description

The present invention relates to the *in vitro* cell culture propagation of hepatitis A virus and, more particularly, to *in vitro* cell culture propagation of hepatitis A virus wherein the inoculum is a human clinical specimen containing the virus.

The present invention provides a method for propagating hepatitis A virus wherein passage of the virus in a susceptible non-human primate prior to *in vitro* cell culture is not required.

According to the present invention a clinical specimen containing hepatitis A virus, e.g. stool extract, saliva, urine, or blood, is used as inoculum to infect a susceptible *in vitro* cell culture. The cell culture may be formed of primary, continuously cultivated, or transformed cells derived from kidney or liver of human or non-human primate origin, specific examples of suitable cell cultures are those derived from fetal or newborn kidney cells from rhesus monkey (FRhK6), cynomolgus monkey, or cercopithecus monkey, or diploid fibroblast cells derived from human or non-human primate lung tissue such as, for example, WI-38 or MRC-5 cell lines. The inoculated cell culture is incubated for an extended period of time until positive results are obtained for the presence of hepatitis A antigen. By extended period of time is meant an incubation of at least about 25 days, and preferably from about 25 up to about 100 days. The incubation is carried out in the presence of a nutrient medium which maintains the cells at temperatures permitting propagation of the virus in the cell culture. Typically, such temperatures are from about 30 to about 39°C. The nutrient medium may be, for example, Eagle's Minimum Essential Medium (EMEM), Williams Medium E, Medium 199, Dulbecco's Modified Eagle's Medium, RPMI Media or Basal Medium Eagle with 0.5% fetal calf serum. The cultures are subsequently harvested and serial passages of the viral agent are carried out. As indicated above the virus may be propagated by serial passages in diploid fibroblast cells derived from human or non-human primate lung tissue. Propagation is effected through at least about 5 serial passages in the diploid fibroblast cells. Preferably propagation is effected through from about 5 to about 50 passages in the diploid fibroblast cells. Most preferably, prior to passage in the diploid fibroblast cells the virus is propagated in a cell-culture derived from primary, continuously cultivated or transformed cells derived from kidney or liver of human or non-human primates. Propagation is effected through from about 5 to about 25 passages in the kidney or liver of human or non-human primates.

This invention allows disease diagnosis by direct virus isolation. Further, it allows the *in vitro* cultivation of the virus in adequate quantity and with appropriate properties for the preparation of human vaccines and diagnostic antigen.

The following examples illustrate the present invention without, however, limiting the same thereto.

### Example 1

A 20 percent extract of human stool in PBS is prepared from acutely ill human hepatitis A patients and clarified by centrifugation. It gives an immune adherence titer of 1:16 for hepatitis A antigen. It is further diluted 1:5 in cell culture medium containing 0.5% fetal calf serum and filtered through a $0.45\mu$ Millipore filter. One ml of this material is inoculated per 25 cm² flask of fetal rhesus kidney (FRhK6) cells. The inoculum is left in place for 4 hours and then removed. Five ml of EMEM containing 0.5% fetal calf serum plus neomycin and glutamine is added per flask and the medium removed and renewed at 5—7 day intervals with incubation at 32—35°C. Coverslips are removed periodically and examined by the direct immunofluorescence technique (Provost et al., P.S.E.B.M., *160*, 213—221, 1979) for hepatitis A antigen. Such examination is essentially negative until day 33 post inoculation, when the first clear positive results are obtained. The cultures are subsequently harvested at day 37 and the cells disrupted by freeze-thawing and sonication. Cellular debris is removed by low speed centrifugation. The supernatant product gives a positive result by radioimmunoassay for hepatitis A antigen.

### Example 2

A second passage of the virus in FRhK6 cells is achieved by inoculating 0.5 ml of the supernatant product from Example 1 into fresh cell cultures, which are handled comparably to those above. Fluorescence antibody examination reveals the first clear evidence of virus presence at day 15 post-inoculation. The cultures are harvested at day 19 post-inoculation and the product gives a positive result for hepatitis A antigen by radioimmunoassay. Additional serial passage of the virus is carried out in cell culture, i.e. passages 3, 4 and 5. By passage 5, heavy virus growth occurs as early as 7—14 days and the virus harvest at day 14 gives strong positive immune adherence (IA) and radioimmune assay (RIA) hepatitis A antigen results.

### Example 3

Antigen, 0.05 ml. obtained as described in Example 2, is incubated with human convalescent hepatitis A sera, 0.02 ml of a 1:20 dilution. The mixture is incubated at 37°C for 1 hour and then held at 4°C for a period of three hours. A drop of the material is placed onto a carbon-coated, 300-mesh copper grid, and allowed to adsorb for 30 seconds. The grid is then stained for 2 minutes with 2% aqueous phosphotungsic acid, pH 6.0 (adjusted with 1N KOH) and examined in a Philips 300 electron microscope at 80KV. After reaction with hepatitis A antibody, characteristics halos of antibody molecules are seen to surround the numerous 27 m$\mu$ hepatitis A virus particles and to bind them into an immune complex.

### Example 4

The final infected cell culture harvests obtained from Example 2, prepared under aseptic conditions, are treated with 1:4000 formalin at 37°C for 72 hours. Excess residual formalin is neutralized with sodium bisulfite. All treatments are performed under aseptic conditions. The product is stored at 4°C Subcutaneous or intramuscular injection of 4 doses of 1 ml given at 2 week intervals into *S. mystax* marmosets and quinea pigs induces circulating hepatitis A antibody in these animals. Further, the marmosets are rendered resistant to challenge with virulent doses of hepatitis A virus.

### Example 5

Hepatitis A virus derived as in Example 1 is serially passaged a total of 15 times in fetal rhesus monkey kidney cells at 35°C. The virus harvest from passage 15 is then successfully propagated in human diploid lung (HDL) cell cultures at 32°C. A total of 5 serial *in vitro* passages of the virus in HDL are carried out. After such passage the hepatitis A virus is attenuated in virulence for both marmosets (*S. labiatus*) and chimpanzees in that intravenous inoculation into these animals produced no serum enzyme elevations (e.g. isocitric dehydrogenase) and only minor changes in liver histopathology while at the same time eliciting a hepatitis A antibody response. All animals inoculated with the attenuated virus resist challenges with 1000 50% infectious doses of virulent hepatitis A virus injected intravenously. Thus the tissue culture-passaged hepatitis A virus constitutes a live, attenuated hepatitis A vaccine.

## Claims

1. A process for propagating human hepatitis A virus *in vitro* in cell culture, characterized in that it comprises inoculating a susceptible cell culture with a human clinical specimen containing hepatitis A virus, incubating the inoculated cell culture for an extended period of time in the presence of a nutrient medium which maintains the cells in culture and at a temperature which permits propagation of the virus until the presence of hepatitis A virus is detected in the cell culture, and harvesting the hepatitis A virus in the cell culture.

2. A process according to claim 1, characterized in that the harvested virus is transmitted serially in cell culture under substantially similar conditions.

3. A process according to claim 1, characterized in that the inoculum is an extract of stool obtained from a human hepatitis A patient during the disease process.

4. A process according to claim 1, characterized in that the cell culture comprises a primary, continuously cultivated or transformed cell derived from kidney or liver of human or non-human primate or diploid fibroblast cells derived from human or non-human primate lung tissue.

5. A process according to claim 4, characterized in that the cell culture comprises cells derived from kidney of fetal or newborn rhesus, cynomolgus, or cercopithecus monkeys.

6. A process according to claim 1, characterized in that the incubation is continued for at least about 25 days.

7. A process according to claim 1, characterized in that the hepatitis A virus is propagated through from about 5 to about 50 serial passages of the virus in diploid fibroblast cells derived from human or non-human primate lung tissue.

8. A process according to claim 7, characterized in that prior to passage in the diploid fibroblast cells, the virus is propagated through from about 5 to about 25 serial passages in non-human primate kidney cells.

9. A process according to claim 7, characterized in that the cell culture comprises any primary or continuously cultivated or transformed cells derived from kidney, liver or lung of human or non-human primates.

10. A process according to claim 9, characterized in that the cell culture comprises monkey kidney cells or diploid lung fibroblast cells of human or non-human primates.

## Revendications

1. Procédé pour propager le virus de l'hépatite A humaine *in vitro* dans une culture cellulaire, caractérisé en ce qu'il implique d'inoculer à une culture cellulaire sensible un spécimen clinique humain contenant le virus de l'hépatite A, faire incuber la culture cellulaire inoculée pendant une durée étendue en présence d'un milieu nutritif qui maintient les cellules en culture et à une température qui permet la propagation du virus jusqu'à ce que la présence du virus de l'hépatite A soit détectée dans la culture cellulaire, et de recueillir le virus de l'hépatite A dans la culture cellulaire.

2. Procédé selon la rev. 1, caractérisé en ce que le virus recueilli est transmis en série dans une culture cellulaire dans des conditions pratiquement analogues.

3. Procédé selon la rev. 1, caractérisé en ce que l'inoculum est un extrait de selle obtenu chez un malade humain atteint d'hépatite A au cours de la maladie.

4. Procédé selon la rev. 1, caractérisé en ce que la culture cellulaire comprend une cellule primaire, continuellement cultivée ou transformée provenant du rein ou du foie d'hommes ou de primates non humains ou des fibroblastes diploïdes provenant de tissu pulmonaire humain ou de primate non humain.

5. Procédé selon la rev. 4, caractérisé en ce que la culture cellulaire comprend des cellules provenant de rein de singes rhésus, macaque cunomolgus ou cercopithèque, foetus ou nouveau-nés.

6. Procédé selon la rev. 1, caractérisé en ce qu'on continue l'incubation pendant au moins environ 25 jours.

7. Procédé selon la rev. 1, caractérisé en ce que le virus de l'hépatite A est propagé par d'environ 5 à environ 50 passages en série du virus dans des fibroblastes diploïdes provenant de tissu pulmonaire humain ou de primate non humain.

8. Procédé selon la rev. 7, caractérisé en ce qu'avant le passage dans les fibroblastes diploïdes, le virus est propagé par d'environ 5 à environ 25 passages en série dans des cellules de rein de primate non humain.

9. Procédé selon la rev. 7, caractérisé en ce que la culture cellulaire comprend n'importe quelle cellule primaire, continuellement cultivée ou transformée provenant du rein, du foie ou du poumon d'êtres humains ou de primates non humains.

10. Procédé selon la rev. 9, caractérisé en ce que la culture cellulaire comprend des cellules de rein de singe ou des fibroblastes pulmonaires diploïdes d'êtres humains ou de primates non humains.

## Patentansprüche

1. Verfahren zum Vermehren von menschlichem Hepatitis-A-Virus in einer Zellkultur in vitro, dadurch gekennzeichnet, daß man eine empfindliche Zellkultur mit einer menschlichen Hepatitis-A-Virus enthaltenden klinischen Probe inokuliert, die inokulierte Zellkultur während eines längeren Zeitraums in Anwesenheit eines Nährmediums, das die Zellkultur erhält, und bei einer Temperatur, die die Vermehrung des Virus ermöglicht, inkubiert, bis die Anwesenheit von Hepatitis-A-Virus in der Zellkultur festgestellt wird, und das Hepatitis-A-Virus in der Zellkultur erntet.

2. Verfahren nach Anspruch 1, dadurch gekennzeichnet, daß das geerntete Virus serienmäßig in eine Zellkultur ünter im wesentlichen gleichen Bedingungen übertragen wird.

3. Verfahren nach Anspruch 1, dadurch gekennzeichnet, daß das Inokulum ein Stuhlextrakt ist, der von einem menschlichen Hepatitis-A-Patienten während der Erkrankung erhalten wurde.

4. Verfahren nach Anspruch 1, dadurch gekennzeichnet, daß die Zellkultur eine primäre kontinuierlich kultivierte oder umgewandelte Zelle, die von der Niere oder der Leber von menschlichen oder nicht-menschlichen Primaten stammt, oder diploide Fibroblastzellen, die von menschlichem oder nicht-menschlichem Primaten-Lungengewebe stammen, umfaßt.

5. Verfahren nach Anspruch 4, dadurch gekennzeichnet, daß die Zellkultur Zellen umfaßt, die von der Niere von fötalen oder neugeborenen Rhesus-, Cynomolgus- oder Cercopithecus-Affen stammt.

6. Verfahren nach Anspruch 1, dadurch gekennzeichnet, daß die Inkubation während mindestens etwa 25 Tagen fortgesetzt wird.

7. Verfahren nach Anspruch 1, dadurch gekennzeichnet, daß das Hepatitis-A-Virus durch etwa 5 bis etwa 50 Serienpassagen des Virus in diploiden Fibroblastenzellen, die von menschlichem oder nicht-menschlichem Primaten-Lungengewebe stammen, vermehrt wird.

8. Verfahren nach Anspruch 7, dadurch gekennzeichnet, daß vor der Passage in den diploiden Fibroblastenzellen das Virus durch etwa 5 bis etwa 25 Serienpassagen in nichtmenschlichen Primaten-Nierenzellen vermehrt wird.

9. Verfahren nach Anspruch 7, dadurch gekennzeichnet, daß die Zellkultur jegliche primären oder kontinuierlich kultivierten oder umgewandelten Zellen umfaßt, die von der Niere, Leber oder Lunge von menschlichen oder nicht-menschlichen Primaten stammen.

10. Verfahren nach Anspruch 9, dadurch gekennzeichnet, daß die Zellkultur Affen-Nierenzellen oder diploide Lungenfibroblastenzellen von menschlichen oder nicht-menschlichen Primaten umfaßt.